# FASCICULE DE BREVET EUROPEEN

(11) **EP 1 122 346 B1**
(45) Date de publication et mention de la délivrance du brevet: **06.05.2004**
(21) Numéro de dépôt: 01420024.0
(22) Date de dépôt: 30.01.2001
(51) Int. Cl.: D03D 15/08

(54) **Tissu élastique pour bande de contention, veineuse notamment**
Elastisches Gewebe für Kompressionsbandage, insbesondere Venöse
Elastic fabric for compression bandage, more especially venous

(30) Priorité: 03.02.2000 FR 0001346
(43) Date de publication de la demande: 08.08.2001
(73) Titulaire: Etablissements Antoine Bertheas & Cie, F-42000 Saint Etienne (FR)
(72) Inventeur: Moulin, Hervé, 42000 Saint Etienne (FR)
(74) Mandataire: Vuillermoz, Bruno

(56) Documents cités:
- EP-A- 0 670 383
- US-A- 3 920 054
- US-A- 4 207 885

## Description

### Domaine Technique

La présente invention concerne un nouveau type de tissu chaîne et trame utilisable plus particulièrement comme bande de contention, veineuse plus particulièrement, présentant une élasticité élevée tant dans le sens long (sens chaîne) que dans le sens travers (sens trame).

Elle a trait plus particulièrement à un nouveau type de bande de contention qui, par rapport à des articles réalisés à ce jour présentant une force de contention équivalente, est beaucoup plus confortable pour l'utilisateur, d'un poids moins élevé et résiste mieux aux lavages successifs auxquels sont soumis de tels articles, et surtout est beaucoup plus stable dimensionnellement, notamment en largeur et dont la force de contention ne se trouve affectée qu'après un nombre beaucoup plus élevé de lavages successifs.

### Techniques antérieures

Dans le domaine médical ou paramédical, les bandes de contention peuvent être classées en deux grandes catégories, à savoir les bandes de contention élastiques dans un seul sens, sens longitudinal en général, utilisées dans le domaine de la confection orthopédique pour réaliser par exemple des genouillères, coudières, ceintures lombaires ainsi que parfois comme bandes de contention veineuse, et les bandes de contention élastique en tous sens, plus particulièrement utilisées dans le domaine de la contention veineuse.

De tels produits doivent répondre à des spécifications précises fonction de leur utilisation, spécifications qui portent sur trois caractéristiques essentielles, à savoir leur capacité d'allongement, la force de contention et leur comportement à l'usage.

Concernant la capacité d'allongement, celui-ci doit être supérieur à 120 % dans le sens longitudinal pour les bandes de contention élastiques uniquement dans le sens longitudinal (sens chaîne), être supérieur à 70 % dans le sens transversal et 120 % dans le sens longitudinal pour les bandes de contention élastiques en tous sens, et supérieur à 100 % dans le sens longitudinal pour les bandes de contention thoraciques ou abdominales.

Concernant la force de contention, les bandes sont classées en quatre grandes catégories, cette force étant déterminée selon un test qui consiste, après trois cycles de fatigue de 0 % à 30 %, à enregistrer alors la force nécessaire pour allonger à 30 % la bande élastique lors du dernier cycle.

A titre indicatif, les bandes de contention sont classées en quatre grandes catégories, à savoir :
- Force 1 : bande présentant une force de contention comprise entre 20 et 45 cN/cm ;
- Force 2 : bande présentant une force de contention comprise entre 46 et 100 cN/cm ;
- Force 3 : bande présentant une force de contention comprise entre 101 et 160 cN/cm ;
- Force 4 : bande présentant une force de contention supérieure à 160 cN/cm.

Dans le domaine de la contention veineuse, les bandes majoritairement utilisées, sont en général de Force 1.

Enfin, de telles bandes doivent présenter de très bonnes caractéristiques de comportement à l'usage, caractéristiques déterminées après une série de tests classiques de lavage, vieillissement artificiel à température de 70°C sous 20 % d'allongement, de tenue en extension pendant 12 heures sous 30 % d'allongement suivi de cinq lavages consécutifs.

Après de tels tests, la perte de force mesurée entre le premier test (après lavage à l'état neuf) et le dernier test, ne doit pas être supérieure à 20 % de la force initiale.

Enfin, la perte de force avant et après stérilisation en chaleur humide à 120°C pendant vingt minutes, ne doit pas être supérieure à 25 % de la force initiale.

A ce jour, pour réaliser de tels articles, on réalise des tissus chaîne et trame qui, d'une manière générale, sont constitués d'une chaîne mixte comportant des fils fortement extensibles permettant de donner l'élasticité aux tissus, et des fils inextensibles, tels que filés de fibres (fibranne ou coton) ou faiblement extensibles, tels que fils multifilamentaires texturés ou non, permettant d'assurer le confort et également le taux de contention que l'on souhaite obtenir.

A titre indicatif, ainsi que cela ressort du document RD-258049 (Research Disclosure), on réalise une telle bande de contention veineuse élastique à partir d'un tissu dont la chaîne est une chaîne mixte comportant des fils fortement extensibles tels que des fils " spandex " du type commercialisé par la Société DU PONT DE NEMOURS sous la marque " *Lycra* ", ces fils fortement extensibles étant, lors du tissage, associés à des fils texturés en Nylon.

Les fils *" Lycra* " sont tissés nus en les maintenant sous tension, chaque fil étant associé avec un fil texturé par passage dans la même dent du peigne.

Dans un tel tissu qui n'est extensible que dans le sens long, la trame est constituée de filés de coton.

Il a également été proposé, notamment comme cela ressort de l'US-A-4 236 550 ou du FR-A-2 716 899 au nom du Demandeur, de réaliser des tissus dont la chaîne est réalisée à partir d'une combinaison de fils fortement élastiques du type " fils guipés " ou " fils à âme " comprenant une âme en caoutchouc naturel ou de préférence en élastofibres, tel qu'élasthanne, associée à des fils de coton, fibranne, ou fils texturés, le tissage étant réalisé dans des conditions telles que les fils non extensibles apparaissent en surface et masquent les fils élastiques qui restent positionnés dans la partie centrale du tissu.

Par ailleurs, ces articles peuvent être bloqués dans le sens trame, auquel cas on utilise comme trame un fil synthétique qui peut être multifilamentaires ou monobrin ou constitué par un filé de fibres.

Lorsque l'on souhaite obtenir une extensibilité en sens travers, on utilise en trame des fils extensibles ou élastiques par nature tels que des fils de caoutchouc (naturel ou à base d'élastomère de synthèse), et plus particulièrement des fils guipés ou des fils à âme.

De tels tissus élastiques, s'ils donnent satisfaction en ce qui concerne le taux de contention qu'ils permettent d'obtenir et le confort pour l'utilisateur, présentent cependant un certain nombre d'inconvénients, à savoir de présenter une résistance à l'abrasion jugée parfois insuffisante et très souvent de ne pas pouvoir être teints en pièce compte tenu de la disparité des constituants entrant dans la composition du tissu.

Enfin, dans le domaine des bandes de contention, il a été proposé de réaliser des articles extensibles non seulement dans le sens long (sens chaîne), mais également dans le sens trame, et ce en utilisant des fils de trame non extensibles en eux-mêmes, tels que par exemple des filés de fibres (coton, fibranne), l'extensibilité transversale étant obtenue grâce au choix d'une armure spécifique de tissage.

Ainsi, le Demandeur commercialise depuis de nombreuses années des bandes de contention dont la chaîne est une chaîne mixte comportant une alternance d'un certain nombre de filés de fibres (fibranne) et de fils élastiques guipés.

Des articles similaires peuvent bien entendu être réalisés avec des filés de coton.

Pour obtenir l'extensibilité dans le sens trame, la disposition du fil de chaîne est telle que deux fils fortement élastiques soient positionnés côte à côte et travaillent avec la trame selon une armure de type " pas de gaze " ou " tour anglais ".

A titre indicatif, de telles bandes de contention comportent, si l'on fait abstraction des zones formant les lisières, un rapport d'enfilage des fils de chaîne comprenant quatre ou cinq filés de fibres non élastiques suivis de deux fils fortement élastiques, ce rapport étant répété **n** fois en fonction de la largeur de la bande à produire.

Dans de tels articles, l'armure " pas de gaze " des fils fortement extensibles modifie le trajet des fils de trame au niveau de la zone de liaison avec des fils de chaîne fortement extensibles, ce qui a pour conséquence d'obtenir une extensibilité dans le sens transversal pouvant atteindre 70 %, allongement requis pour la réalisation de bandes de contention, malgré la présence de trames qui, en elles-mêmes, ne sont pas extensibles.

### Exposé de l'invention

Or on a trouvé, et c'est ce qui fait l'objet de la présente invention, un perfectionnement apporté à ce dernier type d'articles dans lesquels les fils de chaîne élastiques travaillent selon une armure " pas de gaze " ou " tour anglais " qui, par rapport aux articles antérieurs dans lesquels les constituants non élastiques (fils de chaîne, fils de guipage et fils de trame) sont constitués de filés de fibres en fibranne ou coton, présentent un retrait au lavage beaucoup plus faible, conserve ses caractéristiques mécaniques, et notamment un taux de contention non altéré pendant un nombre de lavages beaucoup plus élevé tout en ayant un poids inférieur pour des caractéristiques équivalentes et dont le toucher et le confort pour le patient sont nettement améliorés.

D'une manière générale, la bande de contention conforme à l'invention est donc du type constitué par un tissu chaîne et trame dans lequel la chaîne est une chaîne mixte comportant une alternance de fils non élastiques suivis de deux fils fortement extensibles, ladite séquence étant répétée **n** fois en fonction de la largeur de la bande, les fils de trame étant également des fils non extensibles, ledit tissu présentant une capacité d'allongement tant dans le sens longitudinal que dans le sens transversal, et il se caractérise en ce que :
- les fils fortement élastiques sont des fils guipés constitués d'une âme en élastofibres et d'une couverture constituée d'une ou deux couches de fils synthétiques multifilamentaires texturés par fausse torsion ;
- les fils de chaîne non extensibles et les fils de trame sont des fils synthétiques multifilamentaires texturés, également par fausse torsion ou procédé équivalent, en polyamide notamment ;
- l'armure de tissage est conçue de telle sorte qu'elle comporte, dans un rapport, dans la zone comprise entre les deux lisières, une zone travaillant en armure toile ou dérivé de la toile pour la série de fils synthétiques multifilamentaires texturés, et une zone constituée de deux fils fortement élastiques positionnés côté à côte travaillant quant à eux selon une armure de type " pas de gaze " ou " tour anglais ".

Selon une forme de réalisation préférentielle conforme à l'invention, l'insertion de la trame entre les fils de chaîne est réalisée sous la forme d'une boucle entraînant la dépose de deux bouts consécutifs parallèles dans chaque duite des zones en armure toile (ou multiples de deux pour une armure dérivée de la toile).

Dans ce mode de réalisation, l'armure pas de gaze utilisée pour les deux fils fortement élastiques, est réalisée selon un rapport double de l'armure toile (ou dérivé) permettant ainsi de regrouper, dans chaque « maille » formée par cette armure, deux duites (donc quatre bouts) consécutives.

Par ailleurs, dans l'article conforme à l'invention, les filaments constituant les fils de trame sont des microfilaments dont le titre unitaire est inférieur à un decitex, alors que les fils texturés constituant les fils de chaîne non élastiques et la couverture des fils élastiques sont, quant à eux, des fils conventionnels dont le titre au brin est compris entre 1,5 et 2 dtex.

Enfin, dans cette forme de réalisation préférentielle selon laquelle l'enfilage est tel que le rapport en chaîne est de 5 fils texturés Nylon pour 2 fils fortement élastiques, ce rapport élémentaire est répété deux fois consécutivement avec inversion de l'armure toile de la première séquence à la seconde, conduisant donc à un rapport de base comportant au total dix fils de chaîne.

Dans une telle forme de réalisation, la bande de tissu a un aspect similaire à un " nid d'abeille ".

Grâce à une telle sélection et combinaison de moyens (nature des fils et choix d'une armure de tissage spécifique), on obtient un tissu élastique particulièrement adapté pour réaliser des bandes de contention veineuse, dont le toucher, confort pour l'utilisateur, sont améliorés par rapport aux articles antérieurs dont les fils destinés à assurer le confort et le toucher sont, à ce jour, constitués de filés de coton ou de fibrane.

Par ailleurs, de manière inattendue, il a été constaté que de tels articles étaient beaucoup plus résistants à l'usage que les bandes conventionnelles et que, par ailleurs, l'utilisation de fils texturés par fausse torsion tant en chaîne qu'en trame, permettait, pour un taux de contention équivalent, d'utiliser des fils fortement extensibles en élasthanne de titre inférieur, conduisant donc à une structure beaucoup plus légère.

### Description sommaire des dessins

L'invention et les avantages qu'elle apporte seront cependant mieux compris grâce aux exemples qui suivent et qui sont illustrés par les figures annexées dans lesquelles :
- la figure 1 est une vue schématique, en perspective, montrant la structure générale d'une bande élastique de contention conforme à l'invention ainsi que l'enfilage des fils de chaîne dans les lisses des cadres du métier à tisser ;
- la figure 1a est la représentation graphique de l'armure de tissage ;
- la figure 2 est une reproduction d'une bande de contention réalisée conformément à l'invention.

### Manière de réaliser l'invention

Les bandes de contention élastiques obtenues conformément à l'invention sont réalisées sur un métier à tisser conventionnel pour ce type d'articles, c'est-à-dire un métier dans lequel la trame est insérée entre les fils de chaîne par un ensemble de type " crochet " déplaçable sur toute la largeur de la foule, ce qui entraîne donc la dépose de deux bouts consécutifs, disposés en parallèle dans chaque duite, puisque l'insertion se fait sous la forme d'une boucle.

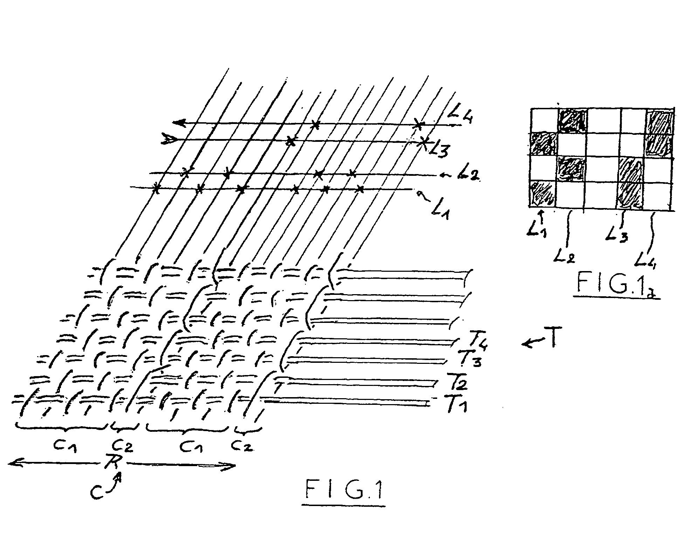

Comme cela est représenté schématiquement à la figure 1, dans le tissu conforme à l'invention, la chaîne (C) est constituée de deux types de fils, à savoir des fils (C1) constitués de fils texturés par fausse torsion et plus particulièrement de fils Nylon séparés entre eux, selon un rythme prédéterminé, par exemple tous les cinq fils tel que cela est représenté à la figure 1, par une paire de fils (C2), fortement élastiques, constitués de fils guipés dont l'âme est un fil élasthanne tel que par exemple un fil commercialisé sous la marque " Lycra " recouvert d'une ou de deux couches de fils de guipage constitués d'un fil texturé par fausse torsion également en Nylon.

Ainsi que cela ressort des figures 1 et 1a, le travail (armure) des fils de chaîne par rapport aux fils de trame est, conformément à l'invention, réalisé de telle sorte que les fils de chaîne (C1) portés par les lisses (L1,L2) travaillent en armure toile avec les trames (T1,T2,T3,T4...), étant rappelé que chaque trame (T1,T2...) est constituée de deux bouts parallèles du même fil puisque ce dernier est déposé à l'intérieur de la foule sous la forme d'une boucle.

Concernant les fils (C2) fortement extensibles, ces deux fils travaillent selon une armure de type " gaze " ou " tour anglais ", c'est-à-dire qu'ils sont monts sur des lisses (L3,L4) déplacées latéralement l'une par rapport à l'autre, et ce selon une séquence bien précise.

Dans la forme de réalisation illustrée, le décalage des lisses (L3,L4) est réalisé toutes les deux duites, c'est-à-dire que deux paires de trame (T1,T2-T3,T4) sont regroupées dans chaque séquence à l'intérieur de la « maille » formée par les deux fils (C2).

Le tissage est réalisé de manière conventionnelle en maintenant la chaîne constituée de fils guipés fortement extensibles sous tension, le renvidage étant, quant à lui, réalisé sans tension.

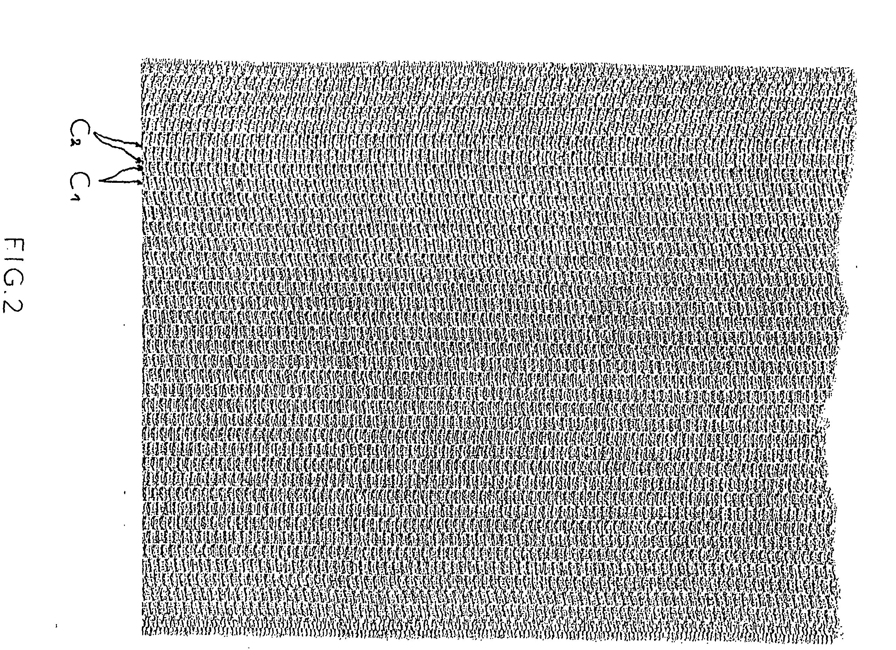

A la tombée du métier, le tissu réceptionné a un aspect tel qu'illustré à la figure 2 et comporte deux lisières parfaitement formées solides, de bonne tenue, la bande réalisée ayant un aspect " nid d'abeille ", résultant de la manière dont est réalisé le tissage et plus particulièrement du choix d'une armure " pas de gaze " pour les fils fortement élastiques (C2), armure dans laquelle deux duites consécutives (4 bouts) sont regroupés dans chaque maille alors que dans les zones constituées de fils texturés, le travail des fils de chaîne est inversé à chaque duite.

Après réalisation, un tel tissu subit un traitement de finition et est notamment teint en pièce, ce qui est rendu possible par le fait que l'ensemble des constituants dudit tissu est à base de fils synthétiques texturés en polyamide.

### Exemple

On réalise un tissu conforme à l'invention du type illustré à la figure 2 à partir d'une armure telle qu'illustrée par la figure 1, et ce dans les conditions suivantes.

### chaîne

. Fils (C1) : fils en Nylon texturés par fausse torsion ayant un titre de 72 dtex/23 brins
. Fils (C2) : fils élastiques comportant une âme en élasthanne ayant un titre de 310 dtex recouvert de deux couches de fils de guipage, orientées en sens inverse l'une de l'autre et constituées d'un fil texturé par fausse torsion en Nylon de titre 1/78 dtex/23 brins

### Trame :

. Fils Nylon texturés par fausse torsion de 2/85 dtex/92 brins.

### Armure de tissage :

. Les fils de chaîne (C) sont délivrés à partir de deux ensouples, l'un pour les fils fortement extensibles (C2), l'autre pour les fils texturés (C1) ;
. les fils de chaîne (C1) sont montés sur deux cadres de lisse (L1,L2) travaillant en armure toile, le nombre total de fils pour une bande ayant une largeur de 10 cm à la tombée du métier étant de 273 ;
. les fils de chaîne fortement extensibles (C2) sont commandés par deux cadres de lisse travaillant selon une armure « pas de gaze », la séquence de commande étant réalisée sur deux duites consécutives ;
. l'enfilage est tel que le rapport en chaîne est de cinq fils texturés Nylon pour deux fils fortement élastiques (C2) répété deux fois avec inversion de l'armure toile de la première séquence à la deuxième. Ce rapport R est répété 18 fois sur la largeur du tissu entre les deux lisières.

### Caractéristiques à la tombée du métier

La bande conforme à l'invention a une largeur de 100 mm et pèse28 g par mètre linéaire.

### Traitement de finition

Le tissu est teint en pièce à 100°C sur une machine conventionnelle à haute température, et ce sans tension, la teinture étant suivie d'un traitement thermique sur rame.

### Caractéristiques du tissu fini selon cet exemple de réalisation

Largeur : 100 mm

Le tissu comporte 19 fils de chaîne par centimètre et 26 trames par centimètre.

Il pèse 28 g par mètre linéaire.

Son taux d'allongement dans le sens long est de 150 % et dans le sens transversal de 65 %.

Sa force de contention mesurée sur un dynamomètre, de manière conventionnelle, en réalisant trois cycles de 0 % à 30 % est de 45 à 55 cN/cm.

Par ailleurs, cette bande présente un faible retrait au lavage, ce retrait étant de l'ordre de 5 % dans le sens transversal après 60 lavages.

En outre, par rapport à un article conventionnel dont les constituants non élastiques sont à base de fibranne ou coton, il présente un temps de séchage à l'air libre beaucoup plus court, dans un rapport de un à deux et, lorsque le séchage est réalisé dans un "tumbler ", de l'ordre de un à trois.

Enfin, il conserve ses caractéristiques de contention malgré les lavages répétés, la perte de force après 40 lavages passant de 50 cN/cm à seulement 42 cN/cm.

A titre indicatif, pour des articles conventionnels présentant un taux de contention équivalent, la perte est de l'ordre de 40 % après 40 lavages, alors que conformément à l'invention, elle n'est que de 16 %. Cette stabilité du taux de contention représente un intérêt tout particulier pour les applications de type contention veineuse.

## Revendications

1. Bande de contention, veineuse notamment, du type constitué par une alternance de fils non élastiques (C1) suivie de deux fils (C2) fortement extensibles" ladite séquence étant répétée **n** fois en fonction de la largeur de la bande, les fils de trame (T) étant également des fils non extensibles, ledit tissu présentant une capacité d'allongement tant dans le sens longitudinal que dans le sens transversal, **caractérisé en ce que** :
- les fils fortement élastiques (C1) sont des fils guipés constitués d'une âme en élastofibres et d'une couverture constituée d'une ou deux couches de fils synthétiques multifilamentaires texturés par fausse torsion ;
- les fils de chaîne (C1) non extensibles et les fils de trame (T) sont des fils synthétiques multifilamentaires texturés, également par fausse torsion ;
- l'armure de tissage est conçue de telle sorte qu'elle comporte, dans un rapport, dans la zone comprise entre deux lisières, une zone travaillant en armure toile ou dérivé de la toile pour la série de fils synthétiques multifilamentaires texturés (C1), et une zone constituée de deux fils fortement élastiques (C1,C2) positionnés côté à côte et travaillant quant à eux selon une armure de type " pas de gaze " ou "tour anglais ".

2. Bande selon la revendication 1, **caractérisée en ce qu'**elle est à base de fils polyamide.

3. Bande selon l'une des revendications 1 et 2, **caractérisé en ce que** l'insertion de la trame (T) entre les fils de chaîne (C) est réalisée la forme d'une boucle entraînant la dépose de deux bouts consécutifs parallèles dans chaque duite des zones en armure toile.

4. Bande selon la revendication 3, **caractérisée en ce que** l'armure « pas de gaze » utilisée pour les deux fils fortement élastiques (C2) est réalisée selon un rapport double de l'armure toile (ou dérivé) permettant ainsi de regrouper, dans chaque " maille " formée par cette armure, deux duites (donc quatre bouts) consécutives.

5. Bande selon l'une des revendications 1 à 4, **caractérisée en ce que** les filaments constituant les fils de trame (T) sont des microfilaments dont le titre unitaire est inférieur à un decitex, alors que les fils texturés constituant les fils de chaîne non élastiques (C1) et la couverture des fils élastiques (C2) sont, quant à eux, des fils conventionnels dont le titre au brin est compris entre 1,5 et 2 dtex.

## Patentansprüche

1. Stütz- oder Kompressionsbinde, insbesondere für venöse Leiden, vom Typ bestehend aus einer Wechselfolge von unelastischen Fäden (C1) gefolgt von zwei stark dehnbaren Fäden (C2), wobei die Folge in Abhängigkeit von der Breite der Binde n Mal wiederholt wird, wobei die Schussfäden (T) ebenfalls nicht dehnbare Fäden sind, wobei das Gewebe eine Dehnungsfähigkeit sowohl in Längsrichtung als auch in Querrichtung aufweist, **dadurch gekennzeichnet, dass**:
- die hochelastischen Fäden (C1) umsponnene Fäden sind, die von einer Seele aus Elastomerfasern und von einem Überzug gebildet sind, welcher aus einer oder zwei Lagen aus synthetischen, falschdraht-texturierten Multifilamentfäden besteht;
- die nicht dehnbaren Kettfäden (C1) und die Schussfäden (T) ebenfalls durch Falschdraht texturierte, synthetische Multifilamentfäden sind;
- die Webbindung derart gestaltet ist, dass sie in einem Rapport in dem Bereich zwischen zwei Webrändern einen in Leinwandbindung oder hiervon abgeleiteter Bindung arbeitenden Bereich für die Reihe synthetischer, texturierter Multifilamentfäden (C1) und einen Bereich enthält, der von zwei hoch elastischen Fäden (C1,C2) gebildet ist, die Seite an Seite positioniert sind und ihrerseits nach einer Gaze- oder Scheindreherbindung arbeiten.

2. Binde nach Anspruch 1, **dadurch gekennzeichnet, dass** sie auf der Basis von Polyamidfäden ist.

3. Binde nach einem der Ansprüche 1 und 2, **dadurch gekennzeichnet, dass** das Eintragen des Schussfadens (T) zwischen den Kettfäden (C) in Form einer Schlaufe erfolgt, was zum Eintrag von zwei aufeinanderfolgenden parallelen Abschnitten in jedem Schuss der Bereiche in Leinwandbindung führt.

4. Binde nach Anspruch 3, **dadurch gekennzeichnet, dass** die Gazebindung, welche für die zwei hoch elastischen Fäden (C2) verwendet wird, nach einem Doppelrapport der Leinwandbindung (oder abgeleitet) vollzogen wird, wodurch es möglich ist, in jeder durch diese Bindung gebildeten "Masche" zwei aufeinanderfolgende Schusslängen (also vier Abschnitte) zu gruppieren.

5. Binde nach einem der Ansprüche 1 bis 4, **dadurch gekennzeichnet, dass** die die Schussfäden (T) bildenden Filamente Mikrofilamente sind, deren Einzeltiter kleiner ist als ein Decitex, während die texturierten Fäden, welche die unelastischen Kettfäden (C1) sowie den Überzug der elastischen Fäden (C2) bilden, ihrerseits herkömmliche Fäden sind, deren Fasertiter zwischen 1,5 und 2 dtex liegt.

## Claims

1. Retaining bandage, in particular venous, the type consisting of an alternation of inelastic threads (C1) which is followed by two highly extensible threads (C2), the said sequence being repeated **n** times as a function of the width of the bandage, the weft threads (T) likewise being non-extensible threads, the said fabric having a capacity for elongation both in the longitudinal direction and in the transverse direction, **characterized in that**:
- the highly elastic threads (C1) are covered threads consisting of a core made from elastofibres and the covering consisting of one or two layers of multifilament synthetic threads textured by false twisting;
- the non-extensible warp threads (C1) and the weft threads (T) are multifilament synthetic threads likewise textured by false twisting;
- the weave is designed in such a way that it comprises in a repeat, in the zone contained between two selvedges, a zone working as a linen or linen derivative weave for the series of textured multifilament synthetic threads (C1) and a zone consisting of two highly elastic threads (C1, C2) positioned side by side and themselves working according to a weave of the "leno" or "English repeat" type.

2. Bandage according to Claim 1, **characterized in that** it is based on polyamide threads.

3. Bandage according to one of Claims 1 and 2, **characterized in that** insertion of the weft (T) between the warp threads (C) is carried out in the form of a loop causing two parallel consecutive ends to be deposited in each pick of the zones consisting of linen weave.

4. Bandage according to Claim 3, **characterized in that** the "leno" weave used for the two highly elastic threads (C2) is produced according to a double repeat of the linen (or derivative) weave, thus making it possible to group two consecutive picks (hence, four ends) in each "stitch" formed by this weave.

5. Bandage according to one of Claims 1 to 4, **characterized in that** the filaments forming the weft threads (T) are microfilaments, the unit linear density of which is lower than one decitex, whereas the textured threads forming the inelastic warp threads (C1) and the covering of the elastic threads (C2) are themselves conventional threads, of which the linear density per strand is between 1.5 and 2 dtex.
